# DEMANDE DE BREVET EUROPEEN

(11) **EP 1 134 249 A1**
(43) Date de publication de la demande: **19.09.2001**
(21) Numéro de dépôt: 01400365.1
(22) Date de dépôt: 12.02.2001
(51) Int. Cl.: C08J 3/075, A61K 7/48

(54) **Composition cosmétique sous forme d'un gel aqueux solide, comprenant un alcool polyvinylique, et procédé de préparation de cette composition**

(30) Priorité: 16.03.2000 FR 0003396
(71) Demandeur: L'OREAL, 75008 Paris (FR)
(72) Inventeur: L'Alloret, Florence, 75013 Paris (FR)
(74) Mandataire: Le Blainvaux Bellegarde, Françoise

(57) **Abrégé**

La présente demande concerne une composition cosmétique comprenant un milieu cosmétiquement acceptable, et se présentant sous forme d'un gel aqueux solide, comprenant par ailleurs au moins un alcool polyvinylique, ladite composition présentant une mesure de contrainte comprise entre 200 et 10 000 Pascals.

L'invention concerne également un procédé de préparation de cette composition, ledit procédé comprenant au moins une étape de refroidissement d'une solution aqueuse homogène d'alcool polyvinylique, jusqu'à une température inférieure ou égale à -5°C, suivi d'au moins une étape de réchauffement de la solution jusqu'à une température de 20-25°C.

## Description

La présente invention a trait à une composition solide, notamment destinée à une application cosmétique, telle qu'une composition de soin ou de maquillage, se présentant plus particulièrement sous forme d'un stick ou d'un bâton.

On connaît dans l'industrie cosmétique de nombreux produits se présentant sous forme solide. On peut notamment citer, par exemple dans le domaine du maquillage, les bâtons ou "sticks" de rouge à lèvres, de fond de teint ou d'ombre à paupières; dans le domaine du soin de la peau ou des lèvres, on peut citer les crayons réparateurs des lèvres, les bâtons ou "sticks" dépigmentants, démaquillants ou hydratants; dans le domaine de l'hygiène, on trouve des sticks déodorants, des sticks ou des pains moussants pour le rasage ou pour le lavage de la peau. Il est en effet particulièrement intéressant de disposer de produits sous forme solide dans la mesure où de tels produits sont très pratiques à utiliser et sont facilement transportables, le produit ne risquant pas de couler.
Ces produits sont assez généralement formulés sur la base d'une part d'une phase grasse pour des raisons de confort et de douceur et d'autre part d'une phase pulvérulente qui apporte la couleur souhaitée, pour les produits de maquillage. Cette phase pulvérulente peut comprendre des pigments et/ou des charges et/ou des nacres. La phase grasse comprend généralement des cires et/ou des huiles et/ou des composés pâteux. Or, les sticks formulés à base de cires peuvent présenter certains inconvénients : ils ont un caractère gras qui n'est pas apprécié par tous les utilisateurs et ils manquent de fraîcheur à l'application. En outre, il est difficile d'y introduire des actifs hydrophiles.
On cherche donc de plus en plus à faire des sticks cosmétiques comprenant une phase aqueuse dans la plus grande concentration possible.
Il est bien sûr connu de faire des sticks non gras tels que les sticks déodorants mais ces derniers sont souvent formulés avec des savons tels que des stéarates de soude ou de triéthanolamine comme agent gélifiant et ils présentent ainsi un pH basique qui peut être agressif pour la peau.
Il est également connu des gels aqueux solides notamment dans les documents WO-A-97/17055 et WO-A-97/17053. Toutefois, ces gels nécessitent l'emploi d'une concentration assez élevée de gélifiant et manquent de fraîcheur et de douceur lors de l'application sur la peau, ou bien font appel à une technique de préparation particulière, l'extrusion et doivent être hydratés au moment de l'emploi. De plus, ces gels deviennent souvent cassants avec le temps.

Aussi, il subsiste le besoin d'un gel aqueux solide, ne présentant pas les inconvénients de l'art antérieur.

La demanderesse a découvert de façon inattendue qu'une composition particulière, comprenant un alcool polyvinylique et susceptible d'être obtenue par un procédé particulier, se présentait sous la forme d'un gel aqueux solide, parfaitement adapté à une utilisation dans le domaine cosmétique.

La présente invention a donc pour objet une composition cosmétique comprenant un milieu cosmétiquement acceptable, et se présentant sous forme d'un gel aqueux solide, comprenant par ailleurs au moins un polymère de type alcool polyvinylique comportant au moins des unités de formule (I): 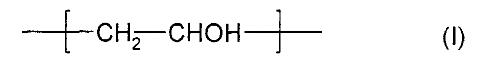 et éventuellement des unités de formule (II): 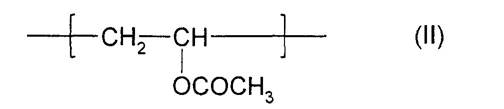 dans lequel les unités de formule (II) sont présentes en une quantité maximum de 5% en mole par rapport au polymère final,
ladite composition présentant une mesure de contrainte comprise entre 200 et 10 000 Pascals (N.m⁻²), de préférence entre 500 et 5000 Pa, et encore mieux entre 1000 et 4000 Pa.

Un autre objet de l'invention est un procédé de préparation d'une composition telle que définie ci-dessus, comprenant au moins une étape de refroidissement d'une solution aqueuse homogène du polymère de type alcool polyvinylique, jusqu'à une température inférieure ou égale à-5°C, suivi d'au moins une étape de réchauffement de la solution jusqu'à une température de 20-25°C.

Un autre objet de l'invention est un procédé de traitement cosmétique d'un support choisi parmi la peau du visage ou du corps, les muqueuses et les fibres kératiniques, comprenant l'application sur ledit support d'une composition telle que définie ci-dessous, ou d'une composition obtenue par le procédé défini ci-après.

Il est connu, par exemple par les brevets GB1342152 et EP107055, de préparer des hydrogels d'alcool polyvinylique par congélation puis décongélation d'une solution aqueuse d'alcool polyvinylique. Ces procédés permettent la préparation d'appâts pour poissons ou de prothèses médicales.
Toutefois, rien dans ces documents ne laissent envisager que les gels ainsi préparés puissent trouver une application toute particulière et très intéressante, dans le domaine cosmétique. Le mérite de cette constatation en revient à la demanderesse, qui a mis au point des gels aqueux solides, présentant des caractéristiques rhéologiques particulières, en choisissant un type bien précis d'alcool polyvinylique, lesdits gels possédant des caractéristiques tout à fait en accord avec un emploi dans le domaine cosmétique.

Notamment, les gels selon l'invention sont stables dans le temps et à la température. Ainsi, après avoir été conservés deux mois à température ambiante ou à 45°C, ils ne présentent aucun phénomène de synérèse (exsudation) ou de déphasage; leur dureté n'a pas varié.
Les gels selon l'invention présentent de bonnes propriétés cosmétiques : notamment ils ne sont pas collants lors de l'application et sont aisés d'utilisation.
Par ailleurs, ils sont particulièrement simples à préparer, notamment par simple mélange des constituants.

La composition selon l'invention comprend donc au moins un polymère de type alcool polyvinylique comportant au moins des unités de formule (I) : 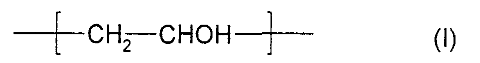 et éventuellement des unités de formule (II): 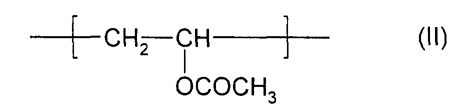 dans lequel les unités de formule (II) sont présentes en une quantité maximum de 5% en mole par rapport au polymère final.
De préférence, les unités de formule (II) sont présentes, dans le polymère final, en une quantité de 0 à 3% en mole, notamment en une quantité de 0,05 à 2% en mole.
Une quantité de 5% en mole par rapport au polymère final d'unités de formule (II) correspond à un degré d'hydrolyse de 95%.
De préférence, ledit polymère a un poids moléculaire moyen en masse (Mw) compris entre 10 000 et 1 000 000 g/mole, notamment de 12 000 à 500 000 g/mole, et encore mieux de 15 000 à 200 000 g/mole.
Comme alcool polyvinylique utilisable selon l'invention, on peut notamment citer ceux vendus sous les dénominations Rhodoviol 4-20, Rhodoviol 8-20 ou Rhodoviol 30-5 par Rhodia, ainsi que Mowiol 4-98, Mowiol 10-98 et Mowiol 20-98 par Clariant.
De préférence, l'alcool polyvinylique peut être présent dans la composition en une quantité de 1 à 50% en poids, de préférence de 5 à 25% en poids, préférentiellement de 7 à 20% en poids, par rapport au poids total de la composition.

La composition comprend par ailleurs une phase aqueuse, qui peut comprendre, outre de l'eau, une eau florale telle que l'eau de bleuet, une eau minérale telle que l'eau de VITTEL, l'eau de LUCAS ou l'eau de LA ROCHE POSAY et/ou une eau thermale.
De préférence, la composition selon l'invention comprend de 50 à 99% en poids, de préférence de 60 à 90% en poids, par rapport au poids total de la composition, d'eau.

La composition selon l'invention comprend un milieu cosmétiquement acceptable, c'est-à-dire un milieu compatible avec une application sur les matières kératiniques telles que la peau, les ongles, les cheveux, les cils et sourcils, les muqueuses et les semi-muqueuses, et toute autre zone cutanée du corps et du visage.
Ce milieu cosmétique acceptable peut comprendre, outre l'eau et l'alcool polyvinylique, les constituants usuellement utilisés dans le type d'application envisagé.

Ainsi, dans un mode de réalisation préféré, la composition selon l'invention comprend par ailleurs au moins un alcool polyhydrique (polyol), notamment de faible poids moléculaire, par exemple de poids moléculaire (Mw) inférieur ou égal à 500.
Les polyols particulièrement préférés sont les alcools possédant de 1 à 18 atomes de carbone et 2 à 6 fonctions hydroxyles, notamment ceux possédant de 2 à 12 atomes de carbone et/ou 2 à 4 fonctions hydroxyles.
On peut en particulier citer, seul ou en mélange, l'éthylène glycol, l'isoprène-glycol, le glycérol, le propane-1,2 diol (propylène glycol), le dipropylène glycol, le diéthylène glycol, le butylène glycol, le pentylène glycol, l'hexylène glycol, la diglycérine, l'érythritol, l'arabitol, l'adonitol, le sorbitol et le dulcitol.
Ledit alcool polyhydrique peut être présent dans la composition à raison de 0 à 20% en poids, de préférence à raison de 0,05 à 15% en poids, notamment à raison de 0,1 à 10% en poids, par rapport au poids total de la composition.

La composition peut également comprendre au moins un poly(oxyalkylène en C2-C5) et plus particulièrement un poly(oxyde d'éthylène) et/ou un poly(oxyde de propylène).
De préférence, ces composés ont un poids moléculaire compris entre 1000 et 6000, notamment entre 1500 et 5000 g/mole.
Ils peuvent être présents dans la composition à raison de 0 à 10% en poids, de préférence à raison de 1-8% en poids, notamment à raison de 2-5% en poids, par rapport au poids total de la composition.

La composition selon l'invention peut également comprendre des solvants autres que l'eau comme par exemple les alcools en C1-C6 tels que l'éthanol et l'isopropanol.

Elle peut aussi comprendre des composés hydrosolubles, notamment des colorants hydrosolubles et/ou des actifs cosmétiques ou pharmaceutiques hydrosolubles.
Parmi les colorants usuels susceptibles d'être employés, on peut citer le sel disodique de ponceau, le sel disodique du vert d'alizarine, le jaune de quinoléine, le sel trisodique d'amarante, le sel disodique de tartrazine, le sel monosodique de rhodamine, le sel disodique de fuchsine, la xanthophylle.
Parmi les actifs hydrosolubles, on peut citer les vitamines hydrosolubles, les filtres solaires hydrosolubles, des principes actifs tels que l'acide hyaluronique et ses dérivés, ou le gluconate de magnésium, des oligo-éléments, des acides aminés, des agents anti-microbiens, des actifs cosmétiques usuels.

Parmi les autres constituants susceptibles d'être incorporés dans la composition, on peut citer des pigments, des nacres et/ou des charges.
Par pigments, il faut comprendre des particules blanches ou colorées, minérales ou organiques, insolubles dans le milieu, destinées à colorer et/ou opacifier la composition. Par nacres, il faut comprendre des particules irisées qui réfléchissent la lumière. Par charges, il faut comprendre des particules incolores ou blanches, minérales ou de synthèse, lamellaires ou non lamellaires, destinées à donner du corps ou de la rigidité à la composition, et/ou de la douceur, de la matité et de l'uniformité au maquillage.
Les pigments peuvent être présents à raison de 0-40 % en poids, par rapport au poids total du gel, de préférence à raison de 0,1 à 30% et de préférence encore à raison de 1-20 %. Ils peuvent être blancs ou colorés, minéraux et/ou organiques, de taille usuelle ou nanométrique. Par taille nanométrique, on entend des pigments dont la taille moyenne des particules va de 5 à 100 nm. On peut citer, parmi les pigments et les nanopigments minéraux, les dioxydes de titane, de zirconium ou de cérium, ainsi que les oxydes de zinc, de fer ou de chrome, les nanotitanes, le bleu ferrique et/ou leurs mélanges. Parmi les pigments organiques, on peut citer le noir de carbone, et les laques couramment employées pour conférer aux lèvres et à la peau un effet de maquillage, qui sont des sels de calcium, de baryum, d'aluminium ou de zirconium, de colorants acides tels que les colorants halogénoacides, azoïques ou anthraquinoniques et/ou leurs mélanges.
Les nacres peuvent être présentes dans le gel à raison de 0-40% en poids, de préférence à raison de 0,1 à 30% et de préférence encore à raison de 1-20% en poids. Parmi les nacres envisageables, on peut citer la nacre naturelle, le mica recouvert d'oxyde de titane, d'oxyde de fer, de pigment naturel ou d'oxychlorure de bismuth ainsi que le mica titane coloré.
Les charges peuvent être présentes dans le gel à raison de 0-60 % en poids, par rapport au poids total du gel, de préférence à raison de 0,1 à 40%, de préférence encore 1-20%, et peuvent être choisies parmi le talc, le mica, la silice, le kaolin, les poudres de Nylon, de poly-β-alanine et de polyéthylène, le Téflon, la lauroyllysine, l'amidon, le nitrure de bore, l'oxychlorure de bismuth, les poudres de polymères de tétrafluoroéthylène, les poudres de polystyrène, les poudres de polyester, les microsphères creuses synthétiques, les microéponges, les microbilles de résine de silicone, les microsphères de silice creuses, les microcapsules de verre ou de céramique.

Par ailleurs, la composition selon l'invention peut comprendre une phase grasse, notamment dispersée dans la phase aqueuse continue. Cette phase grasse peut comprendre des huiles ou des cires d'origine animale, végétale, minérale ou synthétiques; des actifs cosmétiques lipophiles.

La composition selon l'invention peut comprendre en outre tout additif usuellement utilisé dans le domaine considéré, notamment le domaine cosmétique, tel que des antioxydants, des parfums, des huiles essentielles, des conservateurs, des vitamines, des sphingolipides, des composés auto-bronzants tels que la DHA, des filtres solaires, des tensioactifs.
Bien entendu l'homme du métier veillera à choisir ce ou ces éventuels composés complémentaires, et/ou leur quantité, de manière telle que les propriétés avantageuses de la composition selon l'invention ne soient pas, ou substantiellement pas, altérées par l'adjonction envisagée.

La composition selon l'invention se présente sous forme solide. On entend par là qu'on n'observe aucun affaissement de la composition en dehors du récipient la comprenant, en l'absence de stimulation mécanique ou thermique (chauffage notamment).
Au sens de la présente invention, on entend par gel solide, une composition présentant une mesure de contrainte comprise entre 200 et 10 000 Pascals (M.m⁻²), mesurée à température ambiante (20-25°C), après pénétration par un mobile cylindrique plan, en inox, de 6 (ou 12) mm de diamètre, dans la matrice de la composition à une épaisseur de 0,4 mm à une vitesse de 1 mm/s, relaxation pendant 1 seconde et retrait dudit mobile de la matrice de la composition à une vitesse de 1 mm/s, la contrainte étant mesurée avec un analyseur de texture du type "TA-XT2" commercialisé par la Société RHEO.
De préférence encore, la contrainte est comprise entre 500 et 5000 Pa, et encore mieux entre 1000 et 4000 Pa.

La composition présente par ailleurs, de préférence, un module de conservation compris entre 500 Pa et 10 000 Pa, notamment entre 1000 Pa et 5000 Pa.
Elle présente en outre de préférence un module de perte compris entre 50 Pa et 1000 Pa, notamment entre 150 Pa et 500 Pa.
La méthode de mesure de ces modules est décrite avant les exemples.
A partir de ces modules, il est possible de calculer le module complexe G^{*} qui est égal à la racine carrée de la somme des carrés des modules de conservation et de perte.
De préférence, le module G^{*} des compositions selon l'invention est compris entre 200 et 10 000 Pa, notamment entre 1000 et 4000 Pa.

La composition selon l'invention est de préférence préparée par un procédé comprenant au moins une étape de refroidissement d'une solution aqueuse homogène d'alcool polyvinylique, jusqu'à une température inférieure ou égale à -5°C, suivi d'au moins une étape de réchauffement jusqu'à température ambiante (20-25°C).

La solution aqueuse homogène de départ peut être obtenue par tout moyen. On entend par solution homogène, une solution dans laquelle les chaînes d'alcool polyvinylique sont totalement hydratées, ce qui conduit à une solution totalement transparente. On entend par là la définition classique donnée dans le dictionnaire. Ainsi, une composition transparente se laisse aisément traverser par la lumière et permet de distinguer nettement les objets à travers son épaisseur. D'une manière générale, une composition transparente aura une valeur de transmittance maximum de la lumière, quelque soit la longueur d'onde comprise entre 400 et 800 nm, à travers un échantillon de 1 cm d'épaisseur, d'au moins 35%, de préférence d'au moins 50% (voir EP291334). La transmittance peut être mesurée en plaçant un échantillon de 1 cm d'épaisseur dans le rayon lumineux d'un spectrophotomètre travaillant dans les longueurs d'onde du spectre lumineux.
Il est possible de préparer ladite solution homogène par chauffage à une température de l'ordre de 80-90°C d'un mélange eau/alcool polyvinylique.

La solution homogène ainsi obtenue peut être immédiatement refroidie jusqu'à - 5°C ou inférieur, ou peut être laissée à refroidir jusqu'à température ambiante (20-25°C) avant de procéder à l'étape de refroidissement jusqu'à -5°C ou inférieur.
Cette étape de refroidissement peut être effectuées par toute méthode usuelle telle que utilisation d'un bain d'eau glacée, d'azote liquide ou d'un congélateur.
La vitesse à laquelle s'effectue l'étape de refroidissement jusqu'à -5°C ou inférieure, peut être comprise entre 0,1 et 1°C par minute, notamment de l'ordre de 0,3-0,7°C par minute.

Dans un mode de réalisation préféré de l'invention, la solution homogène d'alcool polyvinylique peut être introduite dans un moule, avant d'être refroidie jusqu'à la température souhaitée.

La température à laquelle est refroidie la solution est inférieure ou égale à -5°C, de préférence comprise entre -50 et -10°C, et encore mieux entre -30 et-15°C.
On a constaté que cette température influe sur l'élasticité et la flexibilité du gel obtenu ultérieurement.
Il en est de même de la durée pendant laquelle la solution est maintenue à cette température. Cette durée peut être comprise entre 5 et 48 heures, notamment entre 10 et 30 heures.

La solution refroidie ou congelée, est ensuite réchauffée par tout moyen usuel tel qu'un bain d'huile, jusqu'à une température de l'ordre de 20-25°C, notamment 22-23°C.
La vitesse à laquelle s'effectue l'étape de réchauffement peut être comprise entre 0,1 et 1°C par minute, notamment de l'ordre de 0,2-0,5°C par minute.

On peut alors employer le gel tel quel ou lui faire subir un ou plusieurs autres cycles de refroidissement/réchauffement, aussi appelé cycle de congélation/décongélation.
En effet, on a constaté que le nombre de cycles de congélation/décongélation pouvait influer sur la dureté des gels, ainsi que sur leur stabilité.
Ainsi, on peut laisser le gel obtenu après le premier cycle, pendant 10 minutes à 24 heures, notamment 30 minutes à 12 heures à température ambiante; puis on peut le refroidir à nouveau de la manière et dans les conditions décrites ci-dessus, avant de le réchauffer de la manière et dans les conditions décrites ci-dessus.
On peut ainsi effectuer 2, 3, 4, voire 5 ou 6, cycles de congélation/décongélation.
De préférence, on effectue deux, trois ou quatre cycles de congélation/décongélation.

La composition selon l'invention peut se présenter sous toutes les formes galéniques envisageables, telles que gel aqueux solide; émulsion solide généralement de type huile-dans-eau ou multiple; dispersion d'huile dans l'eau; système multiphases notamment biphase. Elle peut notamment se présenter sous forme de stick ou bâton.
Cette composition trouve notamment une application comme composition de maquillage ou de soin de la peau, en particulier du corps, du visage et/ou du cuir chevelu, ou des fibres kératiniques, en particulier des cheveux, des ongles, des cils et/ou des sourcils, ou encore des muqueuses, en particulier des lèvres. Notamment, elle peut se présenter sous la forme d'une composition d'hygiène corporelle, par exemple sous forme de stick déodorant; d'une composition capillaire, par exemple comme stick de coiffage ou stick de maquillage des cheveux; d'une composition de maquillage de la peau du visage ou du corps, ou des muqueuses, par exemple comme rouge à lèvres, fond de teint coulé en stick ou en coupelle, fard à joues ou paupières, base fixante à appliquer sur un rouge à lèvres classique, stick anti-cernes, brillant à lèvres, eye-liner, mascara, produits de tatouage éphémère; d'une composition de soin de la peau ou des muqueuses, par exemple comme base de soin pour les lèvres, onguent pour le corps, stick de soin; d'une composition solaire ou auto-bronzante.

L'invention est illustrée plus en détail dans les exemples suivants.

### Méthodes de mesure des modules de conservation et de perte

L'appareil employé est un rhéomètre Haake RS 150 en mode dynamique. On effectue un balayage en contrainte en restant dans le domaine de viscoélasticité linéaire. La fréquence est fixée à 1 Hertz.
La géométrie utilisée correspond à un cône-plan de 6 cm (ou de 3,5 cm) de diamètre, formant un angle de 2° avec le plan.

Les modules de conservation et de perte sont déterminés à partir de la déformation du gel soumis à une contrainte sinusoïdale. Les équations classiques de la rhéologie dynamique décrivent la démarche suivie. Le module de conservation correspond à l'énergie emmagasinée par le matériau pendant la déformation tandis que le module de perte correspond à l'énergie dissipée.

### Exemple 1

### Préparation d'une solution aqueuse homogène d'alcool polyvinylique

On prépare une solution aqueuse d'alcool polyvinylique ayant un poids moléculaire de 22 000 g/mole et un degré d'hydrolyse de 97,5-99,5% par mélange de 15g d'alcool polyvinylique dans 85 ml d'eau et chauffage à 85°C pendant 3 heures sous agitation. On obtient, après refroidissement, une solution aqueuse homogène (à 15%) qui sert de base pour les exemples suivants.

### Préparation des gels

On verse la solution précédente dans trois flacons différents, en quantité égale.
- On refroidit la première solution (S1) jusqu'à -20°C à une vitesse de 0,5°C/min, on la maintient à -20°C pendant 23 heures, et on la réchauffe jusqu'à 20°C à une vitesse de 0,3°C/min. Ceci correspond à un seul cycle de congélation/décongélation. On obtient un gel G1.
- On refroidit la seconde solution (S2) jusqu'à -20°C à une vitesse de 0,5°C/min, on la maintient à -20°C pendant 23 heures, et on la réchauffe jusqu'à 22°C à une vitesse de 0,3°C/min; on la maintient 1 heure à 22°C puis on la refroidit à nouveau jusqu'à -20°C à une vitesse de 0,5°C/min, on la maintient à -20°C pendant 23 heures, et on la réchauffe jusqu'à 20°C à une vitesse de 0,3°C/min. Ceci correspond à deux cycles de congélation/décongélation. On obtient un gel G2.
- On refroidit la troisième solution (S3) jusqu'à -20°C à une vitesse de 0,5°C/min, on la maintient à -20°C pendant 23 heures, et on la réchauffe jusqu'à 22°C à une vitesse de 0,3°C/min; on la maintient 1 heure à 22°C puis on la refroidit à nouveau jusqu'à -20°C à une vitesse de 0,5°C/min, on la maintient à -20°C pendant 23 heures, et on la réchauffe jusqu'à 22°C à une vitesse de 0,3°C/min; on la maintient 1 heure à 22°C puis on la refroidit à nouveau jusqu'à -20°C à une vitesse de 0,5°C/min, on la maintient à -20°C pendant 23 heures, et on la réchauffe jusqu'à 20°C à une vitesse de 0,3°C/min. Ceci correspond à trois cycles de congélation/décongélation. On obtient un gel G3.

On mesure la contrainte des gels obtenus (en Pa), après plusieurs jours de conservation à 22°C sous une humidité relative de 50%.

On obtient les résultats suivants :

| | Après 2 jours | Après 6 jours | Après 9 jours | Après 16 jours | Après 32 jours |
|---|---|---|---|---|---|
| Gel G1 (1 cycle) | 310 | 400 | 390 | 510 | 810 |
| Gel G2 (2 cycles) | 980 | 1110 | 1290 | 1680 | 1710 |
| Gel G3 (3 cycles) | 1280 | 1420 | 1620 | 1650 | 1780 |

On constate que le phénomène de gélification se poursuit progressivement au cours du temps.

### Exemple 2

On reprend les trois gels précédents ainsi qu'un quatrième gel G4 obtenu après 4 cycles de congélation/décongélation.
On mesure les modules de conservation et de perte et on calcule le module complexe G^{*} (en Pa).

On obtient les résultats suivants :

| | Après 3 jours | Après 6 jours | Après 12 jours |
|---|---|---|---|
| Gel G1 (1 cycle) | 1050 | 1320 | 1710 |
| Gel G2 (2 cycles) | 2150 | 2750 | 2570 |
| Gel G3 (3 cycles) | 2110 | 2685 | 3980 |
| Gel G4 (4 cycles) | 2665 | 2632 | 3500 |

### Exemple 3

On prépare, selon l'exemple 1, deux solutions d'alcool polyvinylique à 15% dans l'eau, auxquelles on fait subir 1, 2 ou 3 cycles.

On obtient les résultats suivants :

| | Après 1 cycle | Après 2 cycles | Après 3 cycles |
|---|---|---|---|
| PVA de poids moléculaire 22000g/mole, degré d'hydrolyse de 97,5-99,5% | Gel | Gel | Gel |
| PVA de poids moléculaire 15000 g/mole, degré d'hydrolyse de 86-89% | Pas de gel | Pas de gel | Pas de gel |

On constate donc que lorsque le degré d'hydrolyse est trop faible, il n'est pas possible d'obtenir un gel adéquat.

### Exemple 4

On prépare une solution colorée comprenant :
- 10 g d'alcool polyvinylique de PM=22000 et degré d'hydrolyse : 97,5-99,5%
- 5 g de colorant hydrosoluble
- qsp 100 ml d'eau.

On refroidit la solution jusqu'à -20°C à une vitesse de 0,5°C/min, on la maintient à -20°C pendant 23 heures, et on la réchauffe jusqu'à 22°C à une vitesse de 0,3°C/min; ensuite on la refroidit à nouveau jusqu'à -20°C à une vitesse de 0,5°C/min, on la maintient à -20°C pendant 23 heures, et on la réchauffe jusqu'à 20°C à une vitesse de 0,3°C/minute.

On obtient un stick coloré pouvant être employé en cosmétique.

## Revendications

1. Composition cosmétique comprenant un milieu cosmétiquement acceptable, et se présentant sous forme d'un gel aqueux solide, comprenant par ailleurs au moins un polymère de type alcool polyvinylique comportant au moins des unités de formule (I) : 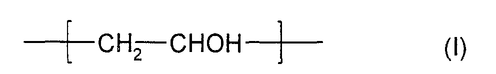 et éventuellement des unités de formule (II): 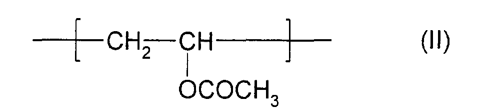 dans lequel les unités de formule (II) sont présentes en une quantité maximum de 5% en mole par rapport au polymère final,
ladite composition présentant une mesure de contrainte comprise entre 200 et 10 000 Pascals (N.m⁻²), de préférence entre 500 et 5000 Pa, et encore mieux entre 1000 et 4000 Pa.

2. Composition selon la revendication 1, présentant par ailleurs au moins l'une des caractéristiques suivantes :
- un module de conservation compris entre 500 Pa et 10 000 Pa, notamment entre 1000 Pa et 5000 Pa.
- un module de perte compris entre 50 Pa et 1000 Pa, notamment entre 150 Pa et 500 Pa.
- un module complexe G^{*} compris entre 200 et 10 000 Pa, notamment entre 1000 et 4000 Pa.

3. Composition selon l'une des revendications précédentes, dans laquelle les unités de formule (II) sont présentes, dans le polymère final, en une quantité de 0 à 3% en mole, notamment en une quantité de 0,05 à 2% en mole.

4. Composition selon l'une des revendications précédentes, dans laquelle le polymère a un poids moléculaire moyen en masse (Mw) compris entre 10 000 et 1 000 000 g/mole, notamment de 12 000 à 500 000 g/mole, et encore mieux de 15 000 à 200 000 g/mole.

5. Composition selon l'une des revendications précédentes, dans laquelle le polymère de type alcool polyvinylique est présent en une quantité de 1 à 50% en poids, de préférence de 5 à 25% en poids, préférentiellement de 7 à 20% en poids, par rapport au poids total de la composition.

6. Composition selon l'une des revendications précédentes, comprenant par ailleurs au moins un alcool polyhydrique (polyol), notamment de poids moléculaire (Mw) inférieur ou égal à 500.

7. Composition selon la revendication 6 dans laquelle l'alcool polyhydrique est choisi parmi les alcools possédant entre 1 et 18 atomes de carbone et 2 à 6 fonctions hydroxyles, notamment ceux possédant entre 2 et 12 atomes de carbone et/ou 2 à 4 fonctions hydroxyles.

8. Composition selon l'une des revendications 6 à 7, dans laquelle l'alcool polyhydrique est présent à raison de 0 à 20% en poids, de préférence à raison de 0,05 à 15% en poids, notamment à raison de 0,1 à 10% en poids, par rapport au poids total de la composition.

9. Composition selon l'une des revendications précédentes, comprenant au moins un poly(oxyalkylène en C2-C5) et plus particulièrement un poly(oxyde d'éthylène) et/ou un poly(oxyde de propylène).

10. Composition selon la revendication 9, dans laquelle le poly(oxyalkylène en C2-C5) est présent à raison de 0 à 10% en poids, de préférence à raison de 1-8% en poids, notamment à raison de 2-5% en poids, par rapport au poids total de la composition.

11. Composition selon l'une des revendications précédentes, comprenant des composés hydrosolubles, notamment des colorants hydrosolubles et/ou des actifs cosmétiques ou pharmaceutiques hydrosolubles.

12. Composition selon l'une des revendications précédentes, comprenant des pigments, des nacres et/ou des charges.

13. Composition selon l'une des revendications précédentes, se présentant sous la forme d'un gel aqueux solide; d'une émulsion solide généralement de type huile-dans-eau ou multiple; d'une dispersion d'huile dans l'eau; d'un système multiphases notamment biphase; d'un stick ou bâton.

14. Composition selon l'une des revendications précédentes, se présentant sous la forme d'une composition d'hygiène corporelle, par exemple sous forme de stick déodorant; d'une composition capillaire, par exemple comme stick de coiffage ou stick de maquillage des cheveux; d'une composition de maquillage de la peau du visage ou du corps, ou des muqueuses, par exemple comme rouge à lèvres, fond de teint coulé en stick ou en coupelle, fard à joues ou paupières, base fixante à appliquer sur un rouge à lèvres classique, stick anti-cernes, brillant à lèvres, eye-liner, mascara, produits de tatouage éphémère; d'une composition de soin de la peau ou des muqueuses, par exemple comme base de soin pour les lèvres, onguent pour le corps, stick de soin; d'une composition solaire ou auto-bronzante.

15. Procédé de préparation d'une composition telle que définie dans l'une des revendications 1 à 14, comprenant au moins une étape de refroidissement d'une solution aqueuse homogène du polymère de type alcool polyvinylique, jusqu'à une température inférieure ou égale à -5°C, suivi d'au moins une étape de réchauffement de la solution jusqu'à une température de 20-25°C.

16. Procédé selon la revendication 15, dans lequel la température à laquelle est refroidie la solution est comprise entre -50 et -10°C, et encore mieux entre -30 et -15°C.

17. Procédé selon l'une des revendications 15 à 16, dans lequel la vitesse à laquelle s'effectue l'étape de refroidissement jusqu'à -5°C ou inférieure, est comprise entre 0,1 et 1°C par minute, notamment de l'ordre de 0,3-0,7°C par minute.

18. Procédé selon l'une des revendications 15 à 17, dans lequel la solution est maintenue à une température inférieure ou égale à -5°C pendant une durée comprise entre 5 et 48 heures, notamment entre 10 et 30 heures.

19. Procédé selon l'une des revendications 15 à 18, dans lequel la solution est réchauffée jusqu'à une température de 22-23°C.

20. Procédé selon l'une des revendications 15 à 19, dans lequel la vitesse à laquelle s'effectue l'étape de réchauffement est comprise entre 0,1 et 1°C par minute, notamment de l'ordre de 0,2-0,5°C par minute.

21. Procédé selon l'une des revendications 15 à 20, dans lequel on effectue 2, 3, 4 voire 5 ou 6 cycles de congélation/décongélation.

22. Procédé selon l'une des revendications 15 à 21, dans lequel on effectue deux, trois ou quatre cycles de congélation/décongélation.

23. Procédé de traitement cosmétique d'un support choisi parmi la peau du visage ou du corps, les muqueuses et les fibres kératiniques, comprenant l'application sur ledit support d'une composition telle que définie dans l'une des revendications 1 à 14, ou d'une composition obtenue par le procédé défini à l'une des revendications 15 à 22.
